# EUROPEAN PATENT APPLICATION

(11) **EP 4 851 284 A1**
(43) Date of publication of application: **09.12.2026**
(21) Application number: 25748640.7
(22) Date of filing: 29.01.2025
(51) Int. Cl.: C12N 15/09, C12N 9/10, C12N 9/22

(54) **RNA-CAPPING ENZYME DERIVED FROM FAUSTOVIRUS S17**

(30) Priority: 30.01.2024 JP 2024011409
(71) Applicant: Takara Bio Inc., Kusatsu-shi Shiga 525-0058 (JP)
(72) Inventor: MATSUMOTO, Hiroyuki, Kusatsu-shi, Shiga 525-0058 (JP); SAGARA, Takehiro, Kusatsu-shi, Shiga 525-0058 (JP); OGAWA, Saori, Kusatsu-shi, Shiga 525-0058 (JP); KUBO, Kaori, Kusatsu-shi, Shiga 525-0058 (JP)
(74) Representative: Müller, Christian Stefan Gerd
(86) International application number: PCT/JP2025/002687
(87) International publication number: WO 2025/164636

(57) **Abstract**

The present invention provides a composition that includes (i) an RNA-capping enzyme derived from Faustovirus S17, (ii) uncapped target RNA, (iii) guanosine triphosphate (GTP), (iv) a methyl group donor, and (v) a buffer. The present invention also provides a target RNA capping method that includes a step for incubating a composition that includes (i) an RNA-capping enzyme derived from Faustovirus S17, (ii) uncapped target RNA, (iii) guanosine triphosphate (GTP), (iv) a methyl group donor, and (v) a buffer.

## Description

### TECHNICAL FIELD

The present invention relates to a composition comprising an RNA capping enzyme derived from Faustovirus strain S17 (hereinafter sometimes referred to as "FCE(S17)").

### BACKGROUND ART

As a new pharmaceutical modality, mRNA drugs have attracted attention. An mRNA drug is a pharmaceutical comprising, as an active ingredient, an mRNA encoding a protein. Upon administration, the mRNA causes the protein to be produced in the body, thereby exerting a medical effect based on the function of the protein. During the global SARS-CoV-2 outbreak in 2020, mRNA vaccines, a type of mRNA drugs, were put into practical use and administered to many people, significantly contributing to infection prevention. mRNA drugs have advantages over other pharmaceuticals in that they allow for the rapid design of vaccines based on the genetic information of viruses and enable the mass production of different types of vaccines using the same manufacturing process.

Eukaryotic mRNA has a modification called a cap at its 5' end. It is believed that caps not only contribute to mRNA stabilization but also enhance translation efficiency by being recognized by translation initiation factors in eukaryotic cells. Therefore, for mRNA drugs, it is highly desirable to add a cap to synthetic RNA.

Currently, two methods are available for RNA capping. In the first method (hereinafter referred to as the "enzymatic RNA capping method"), an RNA capping enzyme is used to convert the target RNA into capped RNA. In the second method (hereinafter referred to as the "co-transcriptional capping method"), a capping analog such as an anti-reverse capping analog (ARCA) is added to an *in vitro* transcription reaction solution. In co-transcriptional capping, the capping analog is co-transcriptionally incorporated into the RNA molecule during *in vitro* transcription.

Compared to the co-transcriptional capping method, the enzymatic RNA capping method provides a higher yield of capped RNA. However, the enzymatic RNA capping method has several drawbacks. For example, the enzymatic RNA capping method requires large amounts of enzymes and purification of the capped RNA from the reaction mixture after capping. Furthermore, conventional RNA capping enzymes have the problem of partially degrading RNA at their optimal temperature (around 37°C).

In the enzymatic RNA capping method, vaccinia virus RNA capping enzyme (hereinafter referred to as "VCE") has been widely used. However, because VCE is a heterodimeric enzyme, it is difficult to manufacture and exhibits unstable enzyme activity. To circumvent these problems, a "single-chain RNA capping enzyme" consisting of a single polypeptide chain has recently been reported.

For example, WO2021/041260 (Patent Document 1) discloses single-chain RNA capping enzymes derived from giant viruses such as Faustovirus, Mimivirus, and Moumouvirus. More specifically, as Faustovirus RNA capping enzymes (hereinafter referred to as "FCE"), FCEs derived from Faustovirus strain D5b, Faustovirus strain E12, Faustovirus strain ST1, and Faustovirus strain LC9 are disclosed.

Furthermore, RNA. 2023 Nov;29(11):1803-1817 (Non-Patent Document 1) demonstrated that an RNA capping enzyme derived from Faustovirus strain D5b exhibited properties advantageous for the production of synthetic mRNA, as compared with VCE.

Furthermore, WO2022/164428 (Patent Document 2) discloses an FCE mutant having an amino acid substitution at a predetermined position in an RNA capping enzyme derived from Faustovirus strain D5b. In this FCE mutant, asparagine (N) at an N-linked glycosylation site is replaced by glutamine (Q), resulting in reduced or absent glycosylation.

### PRIOR ART DOCUMENT

### PATENT DOCUMENT

Patent Document 1: WO2021/041260
Patent Document 2: WO2022/164428

### NON-PATENT DOCUMENT

Non-patent Document 1: RNA. 2023 Nov;29(11):1803-1817

### SUMMARY OF THE INVENTION

### PROBLEMS TO BE SOLVED BY THE INVENTION

To perform enzymatic RNA capping more efficiently, a single-chain RNA capping enzyme with higher enzymatic activity and maintaining high activity even at low temperature is required.

### SOLUTIONS TO THE PROBLEMS

As a result of diligent efforts to solve the above problems, the inventors found that RNA capping enzymes derived from Faustovirus strain S17 have high enzymatic activity and maintain high activity even at low temperature, thus completing the present invention.

In summary, the present invention provides as follows.
[1] A composition comprising:
   (i) an RNA capping enzyme derived from Faustovirus strain S17;
   (ii) an uncapped target RNA;
   (iii) guanosine triphosphate (GTP);
   (iv) a methyl group donor; and
   (v) a buffer.
[2] The composition according to [1], further comprising a nonionic surfactant.
[3] The composition according to [2], wherein the nonionic surfactant is a polysorbate.
[4] The composition according to any one of [1] to [3], wherein the RNA capping enzyme derived from Faustovirus strain S17 is an RNA capping enzyme comprising an amino acid sequence having at least 90% identity to an amino acid sequence set forth in SEQ ID NO: 1.
[5] The composition according to any one of [1] to [3], wherein the RNA capping enzyme derived from Faustovirus strain S17 is an RNA capping enzyme comprising an amino acid sequence having 100% identity to an amino acid sequence set forth in SEQ ID NO: 1.
[6] A method for capping a target RNA, the method comprising a step of incubating a composition comprising:
   (i) an RNA capping enzyme derived from Faustovirus strain S17;
   (ii) an uncapped target RNA;
   (iii) guanosine triphosphate (GTP);
   (iv) a methyl group donor; and
   (v) a buffer.
[7] The method according to [6], wherein the incubating step is carried out at a temperature of 15°C to 35°C.
[8] The method according to [6], wherein the incubating step is carried out at a temperature of 20°C to 30°C.
[9] The method according to any one of [6] to [8], wherein the composition further comprises a nonionic surfactant.
[10] The method according to [9], wherein the nonionic surfactant is a polysorbate.
[11] The method according to any one of [6] to [10], wherein the RNA capping enzyme derived from Faustovirus strain S17 is an RNA capping enzyme comprising an amino acid sequence having at least 90% identity to an amino acid sequence set forth in SEQ ID NO: 1.
[12] The method according to any one of [6] to [10], wherein the RNA capping enzyme derived from Faustovirus strain S17 is an RNA capping enzyme comprising an amino acid sequence having 100% identity to an amino acid sequence set forth in SEQ ID NO: 1.

### EFFECTS OF THE INVENTION

The present invention provides a composition suitable for the enzymatic RNA capping method, and the like.

### BRIEF DESCRIPTION OF THE DRAWINGS

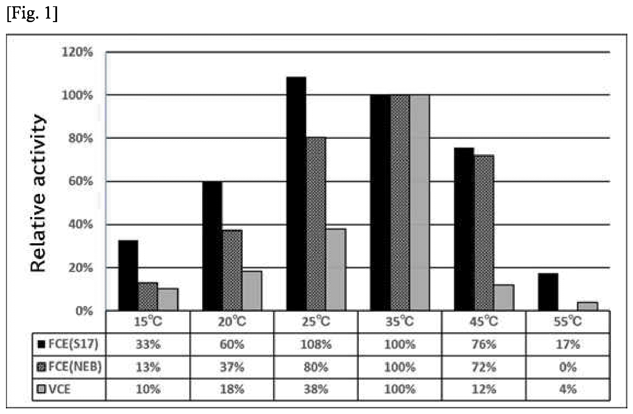

[Fig. 1] Fig. 1 shows relative RNA capping activity of FCE(S17), FCE(NEB), and VCE at each reaction temperature.

### MODE FOR CARRYING OUT THE INVENTION

### (Definition of Terms)

As used herein, the term "RNA capping enzyme" refers to an enzyme having the activity of adding a cap to RNA. More specifically, the term "RNA capping enzyme" refers to a protein that generates a Cap-0 structure through three enzymatic activities: RNA triphosphatase activity, guanylyltransferase activity, and guanine methyltransferase activity.

As used herein, the term "single-chain RNA capping enzyme" refers to an RNA capping enzyme consisting of a single polypeptide chain, having RNA triphosphatase activity, guanylyltransferase activity, and guanine methyltransferase activity. Examples of the single-chain RNA capping enzyme include RNA capping enzymes from Faustovirus, Mimivirus, and Moumouvirus. On the other hand, VCE is a heterodimer and not a single-chain RNA capping enzyme.

### (1) Composition of the present invention

The composition of the present invention is characterized by comprising (i) an RNA capping enzyme derived from Faustovirus strain S17, (ii) an uncapped target RNA, (iii) guanosine triphosphate (GTP), (iv) a methyl group donor, and (v) a buffer.

Faustovirus is a giant virus that was first isolated in 2015. The Faustovirus has a diameter of approximately 0.2 micrometers and contains a 466 kilobase doublestranded DNA genome. Faustovirus infects amoebas. The amino acid sequence of an RNA capping enzyme derived from Fastovirus strain S17 (sometimes referred to as "FCE(S17)") is disclosed in Gene Bank ID: QJX72631.1 (SEQ ID NO: 1).

The RNA capping enzyme used in the present invention may be an enzyme derived from the wild-type Faustovirus strain S17 (SEQ ID NO: 1). Furthermore, the RNA capping enzyme used in the present invention may comprise an amino acid sequence having sequence identity to the amino acid sequence of SEQ ID NO: 1, provided that it is functionally equivalent. The aforementioned function refers to the activity (the activity of adding a cap to RNA) possessed by the RNA capping enzyme derived from Faustovirus strain S17. For example, the RNA capping enzyme used in the present invention has at least 80%, 81%, 82%, 83%, 84%, 85%, 86%, 87%, 88%, 89%, 90%, 91%, 92%, 93%, 94%, 95%, 96%, 97%, 98%, 99%, or 99.5% amino acid sequence identity to the amino acid sequence of SEQ ID NO: 1.

The RNA capping enzyme used in the present invention may comprise an amino acid sequence in which one or several amino acids are substituted, deleted, inserted, or added in the amino acid sequence of SEQ ID NO: 1, provided that it is functionally equivalent. As used herein, the term "one or several" means, for example, the range of 1 to 15, preferably 1 to 10, more preferably 1 to 5, and most preferably 1 to 3.

In one aspect of the present invention, other peptides may be attached to the RNA capping enzyme used in the present invention. Examples of the other peptides include, but are not limited to, signal peptides necessary for the secretion and expression of the enzyme, and affinity tags useful for the purification of the enzyme. The signal peptide may be appropriately selected, depending on the host used for production, from known signal peptides derived from secretory proteins or modified forms thereof, and is typically added to the N-terminus of the RNA capping enzyme to be produced. Furthermore, the affinity tag may be any affinity tag for which a ligand capable of capturing the affinity tag is known, and may be selected from known affinity tags. Examples of the affinity tag include histidine (His) tags, HN tags, HAT tags, glutathione S-transferase (GST) tags, maltose-binding protein (MBP) tags, C-Myc tags, and Strep(II) tag, which consists of eight amino acid residues (Trp-Ser-His-Pro-Gln-Phe-Glu-Lys). These tags may be attached to either the N-terminus or the C-terminus of the RNA capping enzyme being produced. Furthermore, the affinity tag can be removed from the RNA capping enzyme after the purification process is complete. For this purpose, an affinity tag is used that allows for the insertion of a protease recognition sequence, such as Factor Xa, PreScission Protein, thrombin, enterokinase, or TEV protease (Tobacco Etch Virus Protein), between the RNA capping enzyme and the affinity tag. Examples of the RNA capping enzyme used in the present invention include, but not limited to, FCE(S17) having an 8X histidine tag attached to its C-terminus (SEQ ID NO: 3).

The RNA capping enzyme used in the present invention can be manufactured using known recombinant DNA technology. The following explanation is provided with reference to FCE (S17) as an example. Known hosts and vectors can be used. For example, bacteria (such as *Escherichia coli, Bacillus subtilis,* etc.), yeast, filamentous fungi, insect cells, and animal cells (such as mammalian cells including human cells, etc.) may be used as hosts, and a vector suitable for each host may be used. The vector carries a nucleic acid encoding FCE(S17). The nucleic acid may be codon-optimized as needed. FCE(S17) expressed in a host into which the vector containing the nucleic acid has been introduced, or secreted into the culture supernatant of the host, can be purified to the desired purity by a known protein purification method.

The term "cap" refers to a structure found in mRNA of eukaryotic cells and the like, in which a nucleoside is linked to the 5' end of RNA via three phosphate groups. In "capped RNA," the nucleoside is 7-methylguanosine (N7-methylguanosine, sometimes called the standard cap), and is bound to a phosphate group at the 5' position of its ribose portion. The structure of an RNA with a cap may be written as, for example, m⁷GpppN ₁ (pN)_{X}. As used herein, m⁷G represents 7-methylguanosine, ppp represents a triphosphate bridge between the 5' carbon of 7-methylguanosine and the first nucleotide of the RNA produced by transcription, and N₁(pN)_{X} represents the RNA produced by transcription. Furthermore, N₁ represents a nucleotide located at the 5' end of the RNA produced by transcription. The term "p" represents a phosphate group, "G" represents a guanosine nucleoside, and "m ⁷" represents a methyl group at the 7th position of guanine. The cap may have a Cap-0 structure, a Cap-1 structure, or a Cap-2 structure (reviewed in Ramanathan, Nucleic Acids Re., 2016, 44:7511-7526) depending on the enzyme used in the capping reaction and/or the presence of SAM. As used herein, RNA having any cap structure is referred to as "capped RNA".

As used herein, the term "capping" refers to the addition of a cap to the 5' end of RNA. A cap is added to the 5' end of RNA (for example, an uncapped RNA transcript) chemically or enzymatically, independently of transcription, or co-transcriptionally to produce RNA with a 5' cap. As used herein, the cap is added by an enzymatic reaction independent of transcription.

As used herein, the term "uncapped" refers to the state of RNA without a cap structure at its 5' end. Uncapped RNA typically has a triphosphoryl, diphosphoryl, monophosphoryl, or hydroxyl group at its 5' end.

As used herein, the term "target RNA" refers to a polyribonucleotide of interest. The polyribonucleotide may be, for example, a therapeutic RNA or a precursor thereof. The target RNA may originate from intracellular transcription or from in vitro transcription. The target RNA may be present in a mixture, such as an *in vitro* transcription reaction mixture, a cell, or cell lysate. The target RNA may be uncapped.

In some embodiments, the uncapped target RNA may be, for example, 100 nucleotides or longer, and may be an RNA encoding a polypeptide such as a therapeutic protein or a therapeutic vaccine. A target RNA having a secondary structure, including a therapeutic RNA, can be capped more efficiently using the method of the present invention.

The uncapped target RNA can be produced by solid-phase synthesis, or by an *in vitro* transcription reaction to transcribe a DNA template using RNA polymerase. In some embodiments, the RNA produced by an *in vitro* transcription reaction may be purified before capping.

The term *"in vitro"* refers to events that occur not within a multicellular organism, but in an artificial environment such as a test tube, reaction vessel, or cell culture.

The composition of the present invention comprises guanosine triphosphate (GTP). As used herein, the term guanosine triphosphate (GTP) includes modified GTP. Examples of the modified GTP include those having a methyl group at the N7-position of the guanine ring, and those with a label added to the 2- or 3-position of the ribose. In some embodiments, the label attached to the modified GTP may be an oligonucleotide, a detection label such as a fluorophore, or a capture moiety such as biotin or desthiobiotin. In such cases, the label may optionally be linked to the ribose moiety of the nucleotide, for example, via a linker. See, for example, WO2015/085142.

The concentration of guanosine triphosphate (GTP) can be determined by referring to the composition of known reaction solutions. Examples of the concentration of guanosine triphosphate (GTP) contained in the composition of the present invention include, but not particularly limited to, 0.01 mM to 10 mM, preferably 0.05 mM to 5 mM, and more preferably 0.1 mM to 1 mM.

The composition of the present invention comprises a methyl group donor. An example of the methyl group donor is S-adenosylmethionine (hereinafter referred to as "SAM").

The concentration of the methyl group donor can be determined by referring to the composition of known reaction solutions. Examples of the concentration of the methyl group donor contained in the composition of the present invention include, but not particularly limited to, in the case of SAM, 0.001 mM to 10 mM, preferably 0.005 mM to 5 mM, and more preferably 0.01 mM to 0.5 mM.

As used herein, the term "buffer" refers to a reagent that suppresses changes in the pH of a solution when an acid or alkali is added to the solution. Examples of the buffer contained in the composition of the present invention include Tris, HEPES, TAPS, MOPS, Tricin, and MES. A preferred example is Tris. Furthermore, the pH of the composition of the present invention is usually adjusted to a range of 7.0 to 9.0.

The composition of the present invention may further comprise various components such as salts (NaCl, KCl, etc.), metal ions (magnesium ions, etc.), reducing agents (dithiothreitol, etc.), surfactants, and RNase inhibitors. The concentration of these components can be determined by referring to the composition of known reaction solutions.

Examples of the surfactant contained in the composition of the present invention include nonionic surfactants such as polysorbates (polyoxyethylene sorbitan fatty acid esters), Triton X-100 (polyoxyethylene (10) octylphenyl ether), Poloxamer 188, and Nonidet P-40 (octylphenyl-polyethylene glycol), anionic surfactants such as poly(ethylene glycol) 4-nonylphenyl 3-sulfopropyl ether (PNSE), cationic surfactants such as distearyldimethylammonium chloride, and amphoteric surfactants such as cocamidopropyl betaine. Examples of polysorbates include polysorbate 20, polysorbate 21, polysorbate 40, polysorbate 60, polysorbate 61, polysorbate 65, polysorbate 80, polysorbate 81, and polysorbate 85. The composition of the present invention preferably comprises a nonionic surfactant, more preferably a polysorbate, and even more preferably polysorbate 20. Polysorbate 20 is sometimes referred to as "Tween 20" or "polyoxyethylene sorbitan monolurate".

Examples of the concentration of the surfactant contained in the composition of the present invention include, but not particularly limited to, 0.001%(v/v) to 1%(v/v), preferably 0.005%(v/v) to 0.5%(v/v), more preferably 0.01%(v/v) to 0.1%(v/v), and even more preferably 0.02%(v/v).

More efficient capping of an RNA substrate means performing capping using a smaller amount of enzyme added to the capping composition, producing a larger amount of capped RNA (expressed as a percentage of the RNA in the reaction mixture) using the same amount of enzyme, completing the reaction in a shorter period of time, and/or adding a cap more efficiently to RNA having a secondary structure at its 5' end.

The present invention provides a kit for adding a cap to uncapped target RNA. The kit of the present invention is characterized by comprising an RNA capping enzyme derived from Faustovirus strain S17, guanosine triphosphate (GTP), a methyl group donor, and a buffer. The capping enzyme, GTP, methyl group donor, and buffer can be those described above for the composition of the present invention. These components may be packaged individually so that they can be mixed to prepare a reaction mixture at the time of use, or some of the components may be packaged together to facilitate the preparation of a reaction mixture. Examples of the kit of the present invention include a kit comprising a premix solution that forms a complete reaction mixture simply by adding uncapped target RNA and water (e.g., sterile water) at the time of use, and a kit comprising the premix in a dried form. In addition, the kit may further comprise components such as salts (e.g., NaCl and KCl), metal ions (e.g., magnesium ions), reducing agents (e.g., dithiothreitol), surfactants, and RNase inhibitors.

The composition of the present invention can be used for capping a target RNA. The composition of the present invention can be used for capping a target RNA, for example, at low temperature. As used herein, the term "low temperature" refers to a temperature lower than the optimal temperature (around 37°C) of conventional RNA capping enzymes. Non-limiting examples of such a temperature include temperatures of from 10°C to less than 35°C, from 10°C to 30°C, and from 15°C to 25°C.

### (2) Method for capping target RNA of the present invention

The method for capping a target RNA of the present invention is characterized by comprising a step of incubating a composition comprising (i) an RNA capping enzyme derived from Faustovirus strain S17, (ii) an uncapped target RNA, (iii) guanosine triphosphate (GTP), (iv) a methyl group donor, and (v) a buffer.

The method for capping a target RNA according to the present invention can also be achieved by incubating the composition of the present invention.

The incubating step is carried out at a temperature and for a period of time sufficient to form capped target RNA.

The temperature for the incubating step can range from 10°C to 99°C. However, conventional RNA capping enzymes have a problem that RNA partially degrades at their optimal temperature (around 37°C). FCE(S17) is characterized by maintaining the significant capping activity even at low temperature. As used herein, the "low temperature" refers to a temperature lower than the optimal temperature (around 37°C) of conventional RNA capping enzymes. Non-limiting examples of such a temperature include temperatures of from 10°C to less than 35°C, from 10°C to 30°C, and from 15°C to 25°C. Therefore, non-limiting examples of the temperature for the incubating step include 10°C to 55°C, 15°C to 55°C, 10°C to 35°C, 15°C to 35°C, 20°C to 30°C, or 15°C to 25°C. Furthermore, the temperature during the incubating step may be constant or may fluctuate.

The period of time for the incubating step can range from a few seconds to several hours. Non-limiting examples of the period of time for the incubating step include 1 minute to 24 hours, 3 minutes to 12 hours, and 5 minutes to 5 hours.

The method for capping a target RNA according to the present invention may further comprise monitoring the appearance of capped target RNA. For example, the capped RNA can be monitored/detected by denatured urea-polyacrylamide gel electrophoresis, radiometric assay, capillary electrophoresis, or mass spectrometry-based methods.

The target RNA is methylated during the capping reaction using a methyl group donor as a material. When SAM is used as the methyl group donor, S-adenosylhomocysteine (SAH) is produced as a product. The capping activity of the enzyme can be determined by measuring and analyzing the amount of SAH produced using a commercially available methyltransferase activity assay kit.

The method of the present invention generates capped target RNA.

The capped target RNA can be used for various purposes either as it is or in a processed form, for example, encapsulated in a lipid nanoparticle or the like. The capped target RNA may also be formulated into a dosage form suitable for delivery to a subject (e.g., a human). Examples of such formulations may include liquid formulations (e.g., solutions, suspensions, and dispersions), topical formulations (e.g., gels, ointments, drops, and creams), and liposomal formulations.

The formulation may contain pharmaceutically acceptable additives, binders, buffers, coating agents, colorants, release regulators, delivery agents, diluents, disintegrants, dyes, excipients, fillers, lipids, lubricants, salts, and adsorbents.

The capped target RNA may be delivered to eukaryotes. The capped target RNA may be also delivered to animals or animal cells. Examples of the animal include a human, and non-human animals (e.g., a mouse, rat, rabbit, dog, cat, cattle, pig, sheep, horse, and primates). Furthermore, the capped target RNA may be delivered to plants or plant cells to confer or enhance the resistance or tolerance to environmental conditions (e.g., drought, salinity), and/or to prevent, alleviate, or treat herbivory, pathogen infection, or the effects thereof. Furthermore, the capped target RNA may also be delivered to yeast cells.

The capped target RNA and the formulation comprising said RNA may be administered to a subject via any appropriate route of administration, including intra-tissue (e.g., muscle and organs), intravenous, subcutaneous, transdermal, oral, enteral, parenteral, ocular, otic, transmucosal, sublingual, and pulmonary routes (e.g., by spray and/or inhalation), and combinations thereof.

Introduction of the capped target RNA into cells may be performed *in vitro* or *ex vivo.* More specifically, the cells into which the capped target RNA is introduced may be cells forming part of a mammal, cells forming part of a tissue removed from a mammal, or cells isolated from the blood of a mammal.

### EXAMPLES

The present invention will be explained below with reference to Examples, which the present invention is not particularly limited to.

### [Example 1] Preparation of FCE(S17)

The amino acid sequence of an RNA capping enzyme derived from Fastovirus strain S17 (hereinafter referred to as "FCE(S17)") is disclosed in Gene Bank ID: QJX72631.1 (SEQ ID NO: 1). Using this amino acid sequence, a gene encoding FCE(S17) with an 8X histidine tag attached to the C-terminus was chemically synthesized (SEQ ID NO: 2). The obtained synthetic gene was introduced into plasmid pCold IV (manufactured by Takara Bio Inc.) in which the ampicillin resistance gene was replaced with a kanamycin resistance gene, using In-Fusion (registered trademark) HD Cloning Kit (manufactured by Takara Bio Inc.). The amino acid sequence of FCE(S17) with an 8X histidine tag at the C-terminus is shown in SEQ ID NO: 3.

Next, *Escherichia coli* strain BL21 (manufactured by Takara Bio Inc.) was transformed with the plasmid and incubated overnight at 37°C on a 1.5% agarose LB plate containing 50 µg/mL kanamycin. A single colony was selected from the plate, streaked onto a 1.5% agarose LB plate containing 50 µg/mL kanamycin, and incubated at 37°C for 16 to 24 hours. The grown *E. coli* cells were inoculated into an LB medium containing 50 µg/mL kanamycin (hereinafter referred to as "LB-Km medium") and cultured with shaking at 37°C until the OD660 value reached 0.45. A portion of the culture medium obtained by shaking culture was taken, and mixed with 60% glycerol to achieve a final glycerol concentration of 15% to prepare a glycerol stock (hereinafter referred to as "RCB"). One milliliter of RCB was inoculated into 100 mL of LB-Km medium for preculture, and incubated overnight at 37°C with shaking. Two milliliters of the resulting culture medium was inoculated into 200 mL of LB-Km medium for seed culture, and cultured with shaking at 37°C. When the OD600 value reached 0.5, the culture medium was inoculated into 2000 mL of LB-Km medium for the main culture, and cultured at 37°C with stirring. When the OD600 value reached 1.5, IPTG was added to the culture medium to a final concentration of 1 mM, followed by induction culture at 20°C for 18 to 22 hours. The cells were then harvested.

The cells thus obtained were suspended in a solution containing 50 mM Tris-HCl (pH 8.0), 300 mM NaCl, 0.1% Tween 20, 1 mM PMSF, and 5% glycerol in an amount corresponding to five times the cell weight. The suspended cells were disrupted using a high-pressure homogenizer, and then clarified using an MF membrane (0.05 µm, manufactured by Repligen) to remove debris and the like. The clarified solution was then treated with a sterile filtration filter (0.2 µm, manufactured by Sartorius) to obtain a filtrate. To the filtrate, one twenty-fourth volume of a solution containing 50 mM Tris-HCl (pH 8.0), 300 mM NaCl, 0.1% Tween 20, 500 mM imidazole, and 5% glycerol was added and mixed, and then filtered through a bottle-top filter (0.22 µm, manufactured by Corning) to obtain a filtrate. The filtrate was applied to a column packed with Nuvia IMAC Resin (manufactured by BIO RAD) to adsorb the capping enzyme. After washing the column, the adsorbed enzyme was eluted with a buffer containing imidazole. To the resulting eluate, twice volumes of a solution containing 50 mM Tris-HCl (pH 8.0), 300 mM NaCl, 0.1% Tween 20, and 5% glycerol was added and mixed, and then filtered through a bottle-top filter (0.22 µm, manufactured by Corning) to obtain a filtrate. The filtrate was applied to a column packed with Nuvia IMAC Resin (manufactured by BIO RAD) to adsorb the capping enzyme. After washing the column, the adsorbed enzymel was eluted with a buffer containing imidazole. To the resulting eluate, nine volumes of a solution containing 50 mM potassium phosphate buffer (pH 6.5), 0.1 mM EDTA, 1 mM DTT, and 5% glycerol was added and mixed, and then filtered through a bottle-top filter (0.22 µm, manufactured by Corning) to obtain a filtrate. The filtrate was applied to a column packed with Cellufine MAX CM (manufactured by JNC Corporation) to adsorb the capping enzyme. After washing the column, the adsorbed enzyme was eluted with a buffer containing sodium chloride. The resulting eluate was used as FCE(S17) in the following experiments.
SEQ ID NO: 1: MSRRLQRCRDVDQVCEYYNAKGAIGELELRFDKLTPDLFAHVFDKLKPDGEIST 
TMRVSNSDGTAREITFGGGVKTGETFVRKQNICVFDVVDIFSYKVAVSTEETLVD
KPAMEKDASVRFKIRMSVEGAVPNWRIDLTAVKTAELGKIAQHTASLVLQTFPP
NLLKMSGAEVAKLANNSYELELEYIGKTPATKERVDAAAKYAVDLLAGIKNAN
SAVGAVLGESISDICRVAKVIHTPDYATVVCRNPSFKMLLPQVISLTKSSYYGGIY
PPEGMYVAGKTDGVRALVLCEDGVAKVITAESVDITTGTCAGTTILDCELSTGK
SGATLHVFDIIMHNSKPIHTQPFSTRIATDISDVKIPEYKIAIKPFVKIQATALEAAF
KEVYKAPHNEGLILIMDGNDYAMTKTYKWKPLSHNTIDFLIKACPKQLLNIDPY
KPRPGHKLWLLFTTISLDQQRELGIEFIPAWKMLFTDINLFGSRVPIQFMPAINPLA
YICYLPEAATCATGDAINDGDIVEMRAVDGFDTVPKWEPIRVRSDRKDEPGFYG
NNYKIASDIYLNYIDIFQFEDLYKYNPGYFEKNKSDIYVAPNKYRRFLIKNIFSKY
LKNAKWVIDAAAGRGADLHLYKAECVENLLAIDIDPTAISELVRRRNEITGYNR
GHRGHRGGSMRAHMGASHHGAQNCAKSTTLHALVADLRTDPDVLIPKIIQSRP
PERGYDAIVINFAIHYLCDTDEHIRDFLITVSRLLAPNGIFMFTTMDGESIVKLLE
THKVKSGESWTVHTGADDPEAGVVKYSIRRLYDSDKLTKTGQQIAVLLPMSGE
MKTEPLCNIKNIISIARKMGLDLVESADFSVMYEAFARAYPEISARLTPDDKLYN
DLHSYAVFKRKK
SEQ ID NO: 2: 
ATGTCTCGTCGTCTGCAGCGTTGCCGTGACGTTGACCAGGTTTGCGAATACTA
CAACGCTAAAGGTGCTATCGGTGAACTGGAACTGCGTTTCGACAAACTGACC
CCGGACCTGTTCGCTCACGTTTTCGACAAACTGAAACCGGACGGTGAAATCT
CTACCACCATGCGTGTTTCTAACTCTGACGGTACCGCTCGTGAAATCACCTTC
GGTGGTGGTGTTAAAACCGGTGAAACCTTCGTTCGTAAACAGAACATCTGCG
TTTTCGACGTTGTTGACATCTTCTCTTACAAAGTTGCTGTTTCTACCGAAGAA
ACCCTGGTTGACAAACCGGCTATGGAAAAAGACGCTTCTGTTCGTTTCAAAA
TCCGTATGTCTGTTGAAGGTGCTGTTCCGAACTGGCGTATCGACCTGACCGCT
GTTAAAACCGCTGAACTGGGTAAAATCGCTCAGCACACCGCTTCTCTGGTTC
TGCAGACCTTCCCGCCGAACCTGCTGAAAATGTCTGGTGCTGAAGTTGCTAA
ACTGGCTAACAACTCTTACGAACTGGAACTGGAATACATCGGTAAACCCCG
GCTACCAAAGAACGTGTTGACGCTGCTGCTAAATACGCTGTTGACCTGCTGG
 
CTGGTATCAAAAACGCTAACTCTGCTGTTGGTGCTGTTCTGGGTGAATCTATC
TCTGACATCTGCCGTGTTGCTAAAGTTATCCACACCCCGGACTACGCTACCGT
TGTTTGCCGTAACCCGTCTTTCAAAATGCTGCTGCCGCAGGTTATCTCTCTGA
CCAAATCTTCTTACTACGGTGGTATCTACCCGCCGGAAGGTATGTACGTTGCT
GGTAAAACCGACGGTGTTCGTGCTCTGGTTCTGTGCGAAGACGGTGTTGCTA
AAGTTATCACCGCTGAATCTGTTGACATCACCACCGGTACCTGCGCTGGTACC
ACCATCCTGGACTGCGAACTGTCTACCGGTAAATCTGGTGCTACCCTGCACGT
TTTCGACATCATCATGCACAACTCTAAACCGATCCACACCCAGCCGTTCTCTA
CCCGTATCGCTACCGACATCTCTGACGTTAAAATCCCGGAATACAAAATCGCT
ATCAAACCGTTCGTTAAAATCCAGGCTACCGCTCTGGAAGCTGCTTTCAAAG
AAGTTTACAAAGCTCCGCACAACGAAGGTCTGATCCTGATCATGGACGGTAA
CGACTACGCTATGACCAAAACCTACAAATGGAAACCGCTGTCTCACAACACC
ATCGACTTCCTGATCAAAGCTTGCCCGAAACAGCTGCTGAACATCGACCCGT
ACAAACCGCGTCCGGGTCACAAACTGTGGCTGCTGTTCACCACCATCTCTCT
GGACCAGCAGCGTGAACTGGGTATCGAATTCATCCCGGCTTGGAAAATGCTG
TTCACCGACATCAACCTGTTCGGTTCTCGTGTTCCGATCCAGTTCATGCCGGC
TATCAACCCGCTGGCTTACATCTGCTACCTGCCGGAAGCTGCTACCTGCGCTA
CCGGTGACGCTATCAACGACGGTGACATCGTTGAAATGCGTGCTGTTGACGG
TTTCGACACCGTTCCGAAATGGGAACCGATCCGTGTTCGTTCTGACCGTAAA
GACGAACCGGGTTTCTACGGTAACAACTACAAAATCGCTTCTGACATCTACC
TGAACTACATCGACATCTTCCAGTTCGAAGACCTGTACAAATACAACCCGGG
TTACTTCGAAAAAAACAAATCTGACATCTACGTTGCTCCGAACAAATACCGT
CGTTTCCTGATCAAAAACATCTTCTCTAAATACCTGAAAAACGCTAAATGGGT
TATCGACGCTGCTGCTGGTCGTGGTGCTGACCTGCACCTGTACAAAGCTGAA
TGCGTTGAAAACCTGCTGGCTATCGACATCGACCCGACCGCTATCTCTGAACT
GGTTCGTCGTCGTAACGAAATCACCGGTTACAACCGTGGTCACCGTGGTCAC
CGTGGTGGTTCTATGCGTGCTCACATGGGTGCTTCTCACCACGGTGCTCAGA
ACTGCGCTAAATCTACCACCCTGCACGCTCTGGTTGCTGACCTGCGTACCGA
CCCGGACGTTCTGATCCCGAAAATCATCCAGTCTCGTCCGCCGGAACGTGGT
TACGACGCTATCGTTATCAACTTCGCTATCCACTACCTGTGCGACACCGACGA
 
ACACATCCGTGACTTCCTGATCACCGTTTCTCGTCTGCTGGCTCCGAACGGTA
TCTTCATGTTCACCACCATGGACGGTGAATCTATCGTTAAACTGCTGGAAACC
CACAAAGTTAAATCTGGTGAATCTTGGACCGTTCACACCGGTGCTGACGACC
CGGAAGCTGGTGTTGTTAAATACTCTATCCGTCGTCTGTACGACTCTGACAAA
CTGACCAAAACCGGTCAGCAGATCGCTGTTCTGCTGCCGATGTCTGGTGAAA
TGAAAACCGAACCGCTGTGCAACATCAAAAACATCATCTCTATCGCTCGTAA
AATGGGTCTGGACCTGGTTGAATCTGCTGACTTCTCTGTTATGTACGAAGCTT
TCGCTCGTGCTTACCCGGAAATCTCTGCTCGTCTGACCCCGGACGACAAACT
GTACAACGACCTGCACTCTTACGCTGTTTTCAAACGTAAAAAACATCATCAC
CATCATCATCACCACTAA
SEQ ID NO: 3:

MSRRLQRCRDVDQVCEYYNAKGAIGELELRFDKLTPDLFAHVFDKLKPDGEIST
TMRVSNSDGTAREITFGGGVKTGETFVRKQNICVFDVVDIFSYKVAVSTEETLVD
KPAMEKDASVRFKIRMSVEGAVPNWRIDLTAVKTAELGKIAQHTASLVLQTFPP
NLLKMSGAEVAKLANNSYELELEYIGKTPATKERVDAAAKYAVDLLAGIKNAN
SAVGAVLGESISDICRVAKVIHTPDYATVVCRNPSFKMLLPQVISLTKSSYYGGIY
PPEGMYVAGKTDGVRALVLCEDGVAKVITAESVDITTGTCAGTTILDCELSTGK
SGATLHVFDIIMHNSKPIHTQPFSTRIATDISDVKIPEYKIAIKPFVKIQATALEAAF
KEVYKAPHNEGLILIMDGNDYAMTKTYKWKPLSHNTIDFLIKACPKQLLNIDPY
KPRPGHKLWLLFTTISLDQQRELGIEFIPAWKMLFTDINLFGSRVPIQFMPAINPLA
YICYLPEAATCATGDAINDGDIVEMRAVDGFDTVPKWEPIRVRSDRKDEPGFYG
NNYKIASDIYLNYIDIFQFEDLYKYNPGYFEKNKSDIYVAPNKYRRFLIKNIFSKY
LKNAKWVIDAAAGRGADLHLYKAECVENLLAIDIDPTAISELVRRRNEITGYNR
GHRGHRGGSMRAHMGASHHGAQNCAKSTTLHALVADLRTDPDVLIPKIIQSRP
PERGYDAIVINFAIHYLCDTDEHIRDFLITVSRLLAPNGIFMFTTMDGESIVKLLE
THKVKSGESWTVHTGADDPEAGVVKYSIRRLYDSDKLTKTGQQIAVLLPMSGE
MKTEPLCNIKNIISIARKMGLDLVESADFSVMYEAFARAYPEISARLTPDDKLYN
DLHSYAVFKRKKHHHHHHHH



### [Example 2] Effect of capping buffer composition on FCE enzyme activity

Two microliters of an 80-fold dilution of FCE (S17) obtained in Example 1, or 3.1 U of a commercially available FCE RNA capping enzyme (manufactured by New England Biolabs, Cat. No. M2081L), was mixed with 1 µg of 100-base RNA, 0.1 mM GTP, and 0.02 mM SAM in 20 µL of an RNA capping buffer (the composition of which is shown in Table 1). The mixture was incubated at 37°C for 30 minutes. The commercially available FCE [FCE(NEB)] was used based on the activity level indicated on the product.

**[Table 1]**

| Buffer No. | Composition of RNA capping buffer (1X) |
|---|---|
| 1 | 50mM Tris-HCl, 5mM KCl, 1mM MgCl2, 1mM DTT |
| 2 | Buffer No.1 + 0.02% Poloxamer188 |
| 3 | Buffer No.1 + 0.02% Tween20 |
| 4 | Buffer No.1 + 0.02% Poloxamer188 + 0.02% Tween20 |

The nucleotide sequence of 100-base RNA is shown in SEQ ID NO: 4. The 100-base RNA is methylated using SAM as a material during the capping reaction, producing SAH as a product. The amount of SAH produced was measured and analyzed using methyltransferase activity measurement kit MTase-Glo Methyltransferase Assay (manufactured by Promega, model number: V7601) to calculate the capping activity of each enzyme. Furthermore, the relative activity of each enzyme was calculated by setting the activity in Buffer No. 1 to 1. Results are shown in Table 2.

**[Table 2]**

| Buffer No. | Enzyme name | Relative enzymatic activity |
|---|---|---|
| 1 | FCE(S17) | 1.0 |
| | FCE(NEB) | 1.0 |
| 2 | FCE(S17) | 3.6 |
| | FCE(NEB) | 3.6 |
| 3 | FCE(S17) | 5.9 |
| | FCE(NEB) | 4.7 |
| 4 | FCE(S17) | 5.6 |
| | FCE(NEB) | 4.5 |

Both FCE(S17) and FCE(NEB) exhibited activities that were more than 4.5-fold higher in the presence of Tween20 (buffer No. 3 and No. 4) than in the absence of Tween20 (buffer No. 1). These activities were also higher than those observed in the presence of Poloxamer 188 (buffer No. 2). These results show that the addition of Tween 20 to the capping buffer particularly enhances the capping activity of FCE.

SEQ ID NO: 4::
GGGAACCTGTGGCATTTGTGCTGCCGGGAACGGCGTTTCGTGTCTCTGCCGG
TGTGGCAGCCGAAATGACAGAGCGCGGCCTGGCCAGAATGCAAAAGCT

### [Example 3] Enzyme activity of FCE(S17) over a wide temperature range

Ten microliters of a 400-fold dilution of FCE(S17) obtained in Example 1, an amount of FCE(NEB) used in Example 2 corresponding to 3.1 U, or an amount of a commercially available RNA capping enzyme derived from vaccinia virus (VCE; manufactured by Takara Bio, Cat. No. 2460A) corresponding to 2.5 U, was mixed with 1 µg of 100-base RNA, 0.1 mM GTP, and 0.02 mM SAM in 20 µL of an RNA capping buffer (50 mM Tris-HCl, 5 mM KCl, 1 mM MgCl₂, 1 mM DTT, 0.02% Tween20). The mixture was then incubated at a temperature ranging from 15°C to 55°C for 30 minutes. FCE (NEB) and VCE were used based on the activities indicated on the products.

The nucleotide sequence of 100-base RNA is shown in SEQ ID NO: 4. The capping activity of each enzyme was calculated using the method described in Example 2. Furthermore, the relative activity of each enzyme at temperatures ranging from 15°C to 55°C was calculated, with the activity at 35°C defined as 100%. Results are shown in Figure 1.

Whereas FCE(NEB) and VCE exhibited the highest capping activity at 35°C, FCE(S17) exhibited capping activity at 25°C that was comparable to or higher than that at 35°C. Furthermore, FCE(S17) retained higher capping activity than FCE(NEB) and VCE over the low-temperature range (15°C to 25°C).

### INDUSTRIAL APPLICABILITY

The present invention provides a composition suitable and the like for the enzymatic RNA capping method.

### SEQUENCE LISTING FREE TEXT

SEQ ID NO:1: Faustovirus 17S RNA capping enzyme Genbank accession No. QJX72631.1
SEQ ID NO:2: Synthetic gene encoding Faustovirus 17S RNA capping enzyme with 8X histidine tag
SEQ ID NO:3: Faustovirus 17S RNA capping enzyme with 8X histidine tag
SEQ ID NO:4: 100 base RNA

## Claims

1. A composition comprising:
(i) an RNA capping enzyme derived from Faustovirus strain S17;
(ii) an uncapped target RNA;
(iii) guanosine triphosphate (GTP);
(iv) a methyl group donor; and
(v) a buffer.

2. The composition according to claim 1, further comprising a nonionic surfactant.

3. The composition according to claim 2, wherein the nonionic surfactant is a polysorbate.

4. The composition according to any one of claims 1 to 3, wherein the RNA capping enzyme derived from Faustovirus strain S17 is an RNA capping enzyme comprising an amino acid sequence having at least 90% identity to an amino acid sequence set forth in SEQ ID NO: 1.

5. The composition according to any one of claims 1 to 3, wherein the RNA capping enzyme derived from Faustovirus strain S17 is an RNA capping enzyme comprising an amino acid sequence having 100% identity to an amino acid sequence set forth in SEQ ID NO: 1.

6. A method for capping a target RNA, the method comprising a step of incubating a composition comprising:
(i) an RNA capping enzyme derived from Faustovirus strain S17;
(ii) an uncapped target RNA;
(iii) guanosine triphosphate (GTP);
(iv) a methyl group donor; and
(v) a buffer.

7. The method according to claim 6, wherein the incubating step is carried out at a temperature of 15°C to 35°C.

8. The method according to claim 6, wherein the incubating step is carried out at a temperature of 20°C to 30°C.

9. The method according to any one of claims 6 to 8, wherein the composition further comprises a nonionic surfactant.

10. The method according to claim 9, wherein the nonionic surfactant is a polysorbate.

11. The method according to any one of claims 6 to 10, wherein the RNA capping enzyme derived from Faustovirus strain S17 is an RNA capping enzyme comprising an amino acid sequence having at least 90% identity to an amino acid sequence set forth in SEQ ID NO: 1.

12. The method according to any one of claims 6 to 10, wherein the RNA capping enzyme derived from Faustovirus strain S17 is an RNA capping enzyme comprising an amino acid sequence having 100% identity to an amino acid sequence set forth in SEQ ID NO: 1.
